# EUROPEAN PATENT SPECIFICATION

(11) **EP 0 391 972 B1**
(45) Date of publication and mention of the grant of the patent: **15.06.1994**
(21) Application number: 89901429.4
(22) Date of filing: 08.12.1988
(51) Int. Cl.: C12N 15/83, C12N 5/14, A01H 1/00

(54) **CUCUMBER MOSAIC VIRUS COAT PROTEIN GENE**
GEN DES HÜLLPROTEINS DES GURKENMOSAIKVIRUS
GENE DE LA PROTEINE DE L'ENVELOPPE DU VIRUS DE LA MOSAIQUE DU CONCOMBRE

(30) Priority: 21.12.1987 US 135591
(43) Date of publication of application: 17.10.1990
(73) Proprietor: THE UPJOHN COMPANY, Kalamazoo, Michigan 49001 (US)
(72) Inventor: QUEMADA, Hector, D., Kalamazoo - Mi- 49004 (US); SLIGHTOM, Jerry, L., Kalamazoo, MI 49008 (US)
(74) Representative: Perry, Robert Edward
(86) International application number: US8804321
(87) International publication number: WO8905858

(56) References cited:
- EP-A- 0 223 452
- EP-A- 0 279 433
- J. Cell. Biochem. Suppl. 12c, 1988, H. Quemada et al., p. 287, abstract Y 333
- Biological Abstracts, vol. 75, 1983, Philadelphia, PA (US), D.R. Gonsalves et al., abstract 83219
- Gene, vol. 53, nos. 2, 3, 1987, Amsterdam (NL), S.J. Rothstein et al., p. 157
- Chemical Abstracts, vol. 106, 1987, (Columbus, Ohio, US), A.J. Trulson et al., p. 165, abstract 114580b
- Chemical Abstracts, vol. 99, 1983, (Columbus, Ohio, US), M. Edwards et al., p. 431, abstract 191204w
- Biological Abstracts/RRM, (Philadelphia, PA, US), D.K. Lakshman et al., abstract 6536

## Description

This invention relates to a coat protein gene of cucumber mosaic virus strain c (CMV-C). More specifically the invention relates to a process for preparing said gene as well as its incorporation into a transfer vector and to its use to produce transformed plant cells and transformed plants which are resistant to CMV viral infections.

### BACKGROUND OF THE INVENTION

Cucumber mosaic virus (CMV) is a single-stranded (+) RNA plant virus which has a functionally divided genome. The virus genome contains four RNA species designated RNAs1-4; 3389 nucleotides (nt), 3035 nt, 2193 nt and 1027 nt, respectively (Peden and Symons, 1973; Gould and Symons, 1982; Rezaian et al., 1984; Rezaian et al., 1985). Only RNAs1-3 are required for infectivity (Peden and Symons, 1973) because the coat protein, which is encoded by RNA 4, is also encoded by RNA 3. Translations of CMV RNAS yield a 95KDal polypeptide from RNA 1, a 94kDal polypeptide from RNA 2, (Gordon et al., 1983) and two polypeptides from RNA 3: its 5' end encodes a 35KDal polypeptide, and its 3'' end encodes a 24.5kDal polypeptide (Gould and Symons, 1982). The 24.5kDal polypeptide is identical to that encoded by RNA 4 and is the coat protein.

The CMV coat protein gene does not contain the signals necessary for its expression once transferred and integrated into a plant genome. It must be engineered to contain a constitutive promoter 5' top its translation initiation codon (ATG) and a poly(A) addition signal (AATAAA) 3' to its translation termination codon. Several promoters which function in plants are available, but we believe that the best promoters are the constitutive promoters from CaMV, the Ti genes nopaline synthase (Bevan et al.,1983) and octopine synthase (Depicker et al., 1982), and the bean storage protein gene phaseolin. The poly (A) addition signals from these genes are suitable for our purposes as well.

### INFORMATION DISCLOSURE

Plants that are resistant to virus diseases and methods for producing them are described in EP 223,452.

An, G., et al. (1985) "New Cloning Vehicles for Transformation Of Higher Plants". EMBO J. 4:277-284; Dodds, J.A., et al. (1985) Cross protection between strains of cucumber mosaic virus: effect of host and type of inoculum on accumulation of virions and double-stranded RNA of the challenge strain. Virology 144:301-309. Pietrzak, M., et al. (1986) Expression in plants of two bacterial antibiotic resistant genes after protoplast transformation with a new plant expression vector. Nuc. Acids Res 14:5857-5868.

### SUMMARY OF THE INVENTION

This invention provides: The coat protein gene from the C strain of cucumber mosaic virus (CMV-C).

A plant transformation vector comprising the coat protein gene from CMV-C, the promoter of the 35S gene of cauliflower mosaic virus and the polyadenylation signal of either the cauliflower mosaic virus 35S gene or the phaseolus vulgaris seed storage protein gene.

A bacterial cell containing a plant transformation vector comprising the coat protein gene from CMV-C, the 35S promoter of cauliflower mosaic virus and the polyadenylation signal of either the cauliflower mosaic virus 35S gene or the Phaseolus vulgaris seed storage protein gene.

A transformed plant cell containing the coat protein gene from CMV-C, the cauliflower mosaic virus 35S promoter and the polyadenylation signal of either the cauliflower mosaic virus gene or the Phaseolus vulgaris seed storage protein gene (phaseolin).

A plant comprising transformed cells containing the coat protein gene of CMV-C, the cauliflower mosaic virus 35S promoter and the polyadenylation signal of either the cauliflower mosaic virus gene or the Phaseolus vulgaris seed storage protein gene (phaseolin). Transformed plants of this invention include beets, citrus fruit, corn, cucumber, peppers, potatoes, soybean, squash and tomatoes. Especially preferred are members of the cucurbitaceae (squash, cucumber, i.e.,) and solanaceae (peppers, tomatoes, i.e.) family.

A process for producing virus-resistant plants comprising propagating a plant expressing the coat protein gene from the C strain of cucumber virus. Especially preferred is the process for producing members of the cucurbitacaea and solanacaed families.

### DESCRIPTION OF THE PREFERRED EMBODIMENT

Charts 1 to 5 are set forth to illustrate the constructions of this invention. Certain conventions are used to illustrate plasmids and DNA fragments as follows:
(1) The single line figures represent both circular and linear double-stranded DNA.
(2) Asterisks (*) Indicate that the molecule represented is circular. Lack of an asterisk indicates the molecule is linear.
(3) Junctions between natural boundaries of functional components are indicated by vertical lines along the horizontal lines.
(4) Genes or functional components are indicated.
(5) Distances between genes and restriction sites are not to scale. The figures show the relative positions only unless indicated otherwise.

Most of the recombinant DNA methods employed in practicing the present invention are standard procedures, well known to those skilled in the art, and described in detail, for example, EP-223452 which is incorporated herein by reference. Enzymes are obtained from commercial sources and are used according to the vendor's recommendations or other variations known to the art. Reagents, buffers, and culture conditions are also known to those in the art. General references containing such standard techniques include the following: R.Wu, ed. (1979) Methods in Enxymology Vol.68; J.H. Miller (1972) Experiments in Molecular Genetics; T. Maniatis et al.(1982) Molecular Cloning: A Laboratory Manual; and D.M. Glover, ed. (1985) DNA Cloning Vol II, all of which are incorporated by reference.

### Example 1 Isolation of CMV RNAs

Cucumber mosaic virus strain C (CMV-C) was propagated in tobacco plants and RNA was isolated by the method of Lot et al. (Annals of Phytopathology 4:25, 1972).

### Example 2 Cloning of CMV-C

### (a) Synthesis of double-stranded cDNA3

Total CMV-C RNA was polyadenylated in order to provide a site for the annealing of an oligo dT primer. The reaction buffer was as follows: 5 »l, 1 M Tris pH 7.9; 1 »l, 1 H MgCl₂; 2.5 »l, 0.1 M MnCL₂; 5 »l, 5 » NaCl; 0.5 »l, 100 mM ATP; 18 »l, 2.8 mg/ml bovine serum albumin. 3.2 »l of this buffer were mixed with 2 »g of CMV-C total RNA. 3.8»l H₂O and 1 »l of poly-A polymerase were added, and the reaction mixtures were incubated at 37°C. for 10 minutes.

The resulting polyadenylated RNA was used in the cDNA synthesis protocol of Polites and Marotti (Biotechniques 4:514, 1986), except that 0.75 mM KCl was used instead of 50 mM NaCl, and 130 uCi/100 »l ³²P-dCTP was used instead of 10-50 uCi/100 »l^{.}

After ds-cDNA was synthesized, it was purified by G-100 column chromatography, precipitated with ethanol, and suspended in 20 »l of 1X Eco R1 methylase buffer (100 nM NaCl, 100 mM Tris-HCL pH 8.0, 1 mM EDTA, 80 »M S-adenosyl methionine, 100 »g/ml bovine serum albumin). After removal of a 2 »l aliquot for subsequent gel analysis, an additional 1 »l of 32 mM S-adenosyl methione was added to the reaction mixture mix, and 1 »l (20 units) of Eco RI methylase. The reaction was incubated at 37°C. for 30 minutes and stopped by incubation at 70°C. for 10 minutes.

Two »l were removed from the above reaction, and 1 »l (5 units) of E. coli DNA polymerase I klenow fragment was added. The reaction was incubated at 37°C. for 10 minutes, then extracted with phenol/chloroform before precipitating with ethanol. The pellet was washed in 70% ethanol, then in 70% ethanol/0.3 M sodium acetate.

The pellet was dried and resuspended in 8 »l 0.5 »g/»l phosphorylated Eco RI linkers (available from Collaborative Research, Inc, 128 Spring Street, Lexington, MA 02173). One »l 10X ligase buffer (800 mM Tris-HCl pH 8.0, 200 mM MgCl₂, 150 mM DTT, 10 mM ATP) and 1 »l of T4 DNA ligase (4 units/»l) were added, and the reaction was incubated overnight at 15°C.

The ligation reaction was then stopped by incubation at 65°C. for 10 minutes. Sixty »l of water, 10 »l of 10X Eco RI salts(900 mM Tris pH 8.0, 100 mM MgCl2, 100 mM NaCl), and 10 »l of EcoRI (10 units/»l) were added, and the reaction was incubated at 37°C. for 1 hr (a 5 »l aliquot was removed at the beginning for subsequent gel analysis). The reaction was stopped by phenol/chloroform and chloroform extraction. A 5 »l aliquot was removed for gel analysis, and half of the remainder was frozen for future use. The other half was purified by G-100 column chromatography. The G-100 fractions containing the cDNA were concentrated by butanol extraction, precipitated with ethanol, and resuspended in 10 »l of H₂O. After removing 3 »l for subsequent analysis, 1 »l lambda gtll arms (available from Stratagene Co., 3770 Tandy St, San Diego, Ca. 92121), 1 »l of 10X ligase buffer, and 1 »l T4 DNA ligase were added, and the reaction was incubated at 15°C. overnight.

The resulting ligated lambda gtll/cDNA molecules were packaged according to the procedure recommended by the manufacturer of the packaging extract (Gigapack plus, also from Stratagene). This yielded recombinant lambda phage, which were plated according to methods known to those skilled in the art.

Lambda clones containing the coat protein gene were identified by hybridization with radioactively labelled single-stranded cDNA from purified RNA 4 of the whiteleaf strain of CMV (obtained from Dr. D. Gonsalves, Cornell University, Geneva, NY). This RNA 4 single-stranded cDNA was synthesized as follows: RNA 4 molecules were polyadenylated as described above for total CMV-C RNA, except that 5.8 »g RNA 4 was used. First strand synthesis was as described by Polites and Marotti (Biotechniques 4:514, 1986) except that non-radioactive dCTP was not included. Instead, 260 uCi/100 »l of radioactive dCTP was used.

The labelled single-stranded cDNA was purified by P6 column chromatography and used to probe replicate filters lifted from the lambda phage plates mentioned above. The single-stranded cDNA hybridized with DNA from several phage clones, indicating that they contained at least a part of the CMV-C coat protein gene. Several of these lambda clones were grown, and DNA from them was isolated according to methods known to those skilled in the art.

### Example 3 Construction of a pUC19 Clone containing the CMV-C coat protein gene

Several of the cloned cDNA's mentioned in the above example were transferred to the plasmid vector, pUC19 (available from Bethesda Research, P.O. Box 6009, Gaithersburg, Md 20877), using standard methods. The cloned fragments in pUC19 were then sequenced by the technique described by Maxam and Gilbert (Methods in Enzymology 65:499, 1980). Based on this information, one clone was identified as containing the entire coat protein gene. The sequence of this clone (designated pCMV9.9) is shown in Chart 1.

### Example 4 Construction of a micro T-DNA plasmid containing a plant-expressible CMV-C coat protein gene with the CaMV 35S polyadenylation signal

In order to attach the CaMV 35S promoter and polyadenylation signal, a fragment extending from the Acc I site (at position 311) to the Eco RI site (at position 1421) was removed from pCMV9.9 and ligated into the multiple cloning site of the vector pDH51 (Pietrzak et al., 1986) (available from Thomas Hohn, Friedrich Miescher Institute, P.O. Box 2543, CH-4002, Basel, Switzerland). This was accomplished by complete Eco RI and partial Acc I digestion of pCMV9.9, creating a blunt-ended molecule out of the appropriate Acc I-Eco RI fragment (using the Klenow fragment of E. Coli DNA polymerase I), and ligating it into the Sma I site of pDH51 Chart 2). This clone, designated pDH51/CP19, was sequenced by the Maxam-Gilbert technique to confirm its suitability for expression in plants.

The plant expressible coat protein gene was then moved into a vector suitable for Agrobacterium-mediated gene transfer. An Eco RI-Eco RI fragment was removed from pDH51/CP19 and placed into the Eco RI site of the plasmid, pUC1813 (available from Robert Kay, Dept. of Chemistry, Washington State University, Pullman, Washington), creating the plasmid pUC1813/CP19. A 1.8 k.b. fragment containing the plant expressible gene was removed by partial Hind 3 digestion and ligated into the Hind 3 site of the vector, pGA482 (An et al., 1985) (available from Gynehung An, Institute of Biological Chemistry. Washington State University).

The resulting plasmid was designated pGA482/CP19H (Chart 3). The plant expressible gene for glucuronidase (available from Clontech Laboratories, Inc., 4055 Fabian Way, Palo Alto, CA) was then added to pGA482/CP19H by removing a partial Bam HI-Bam HI fragment from a pUC1813 clone containing the gene and inserting it into the Bgl II site of pGA482/CP19. This final construction was designated pGA4-82/CP19/GUS (Chart 3) and was confirmed by Maxam-Gilbert sequencing.

This plasmid, or its derivatives, can be transferred into Agrobacterium strains A208, C58, LBA4404, C58Z707, A4RS, A4RS(pRi-278b) and others. Strains A208, C58, LBA4404, and A4RS are available from ATCC, 12301 Parklawn Drive, Rockville, MD. A4RS(pRi278b) is available from Dr. F. Casse-Delbart, C.N.R.A., Routede Saint Cyr, F78000, Versailles, France. C58Z707 is available from Dr. A.G. Hepburn, University of Illinois, Urbana, IL.

### Example 5 Construction of a micro T-DNA plasmid containing a plant-expressible CMV coat protein gene with the phaseolin polyadenylation signal

The 35S polyadenylation signal was replaced by that of phaseolin by removing an Avr II-Xba fragment from pDH51/CP19. This digestion removes a DNA region which includes the 3' terminus of the coat protein clone (however, all of the CMV coat protein coding region remains intact), an artificially introduced stretch of A-residues, and part of the pDH51 polylinker. An Xba I-Xba I fragment from a pUC1813 clone containing the phaseolin polyadenylation signal was used to replace the Avr II-Xba I fragment. This construction was then removed by digestion with Eco-RI, and cloned into the Eco RI site of pUC1813. This pUC1813 clone was then digested with Xba I, and the fragment consisting of the 35S promoter, the coat protein coding region, and the phaseolin polyadenylation signal was ligated into Xba I site of pGA482. The glucuronidase gene was then added as described above to produce the plasmid pGA482/CP19A-/411GUS (Chart 4). The structure of the plasmid was confirmed by Maxam-Gilbert sequencing.

An alternative plasmid (designed to test the effect that the stretch of A-residues have on expression) was constructed by placing the phaseolin polyadenylation signal from pUC1813 (see above) into the Xba I site of pDH51/CP19. A fragment containing the 35S promoter, coat protein coding sequence, and the phaseolin and 35S polyadenylation signals were then removed from pDH51/CP19 by Eco RI digestion, and ligated into pUC1813. A fragment containing the 35S promoter, coat protein coding sequence (including the artificially synthesized stretch of A-residues), and the phaseolin polyadenylation signal was then removed from that plasmid by partial Xba I digestion and ligated to the Xba-I site of pCA482. The glucuronidase gene was subsequently inserted as described above. The resulting plasmid was designated pGA482/CP19/411/GUS (Chart 5), and the structure was confirmed by Maxam-Gilbert sequencing.

These plasmid, or their derivatives, are transferred into Agrobacterium strains A208, C58, LBA4404, C58Z707, A4RS, A4RS(pRi-278b) and others which in turn are utilized to insert the CMV-C coat protein genes into plant cells by methods described in Example 4 and Chart 3 of U.S Patent application Serial No. 135,655 filed December 21, 1987, entitled "Agrobacterium Mediated Transformation of Germinating Plant Seeds". The Example and Chart are appended hereto as Exhibit A.

### EXHIBIT A

### Example 3

The purpose of this example is to incorporate a modified seed storage protein which encodes a higher percentage of sulfur-containing amino acids; such a gene is referred to as High Sulfur Storage Protein (HSSP)-gene. This gene is constructed so that it is developmentally expressed in the seeds of dicotyledonous plants; this has been accomplished by using the phaseolin promoter. The modified gene must encode a substantial number of sulfur-containing amino acids. Naturally occurring HSSP-genes can also be used. The two best naturally occurring HSSP-genes are the beta zein gene (15 kD) (Pedersen et al (1986) J. Biol. Chem. 201:6279) and the Brazil nut protein (Altenbach et al. (1987) Plant Mol. Bio. 8:239). However, any other natural or synthetic gene derivative of an HSSP-gene can be used for the improvement of the nutritional value of seeds.

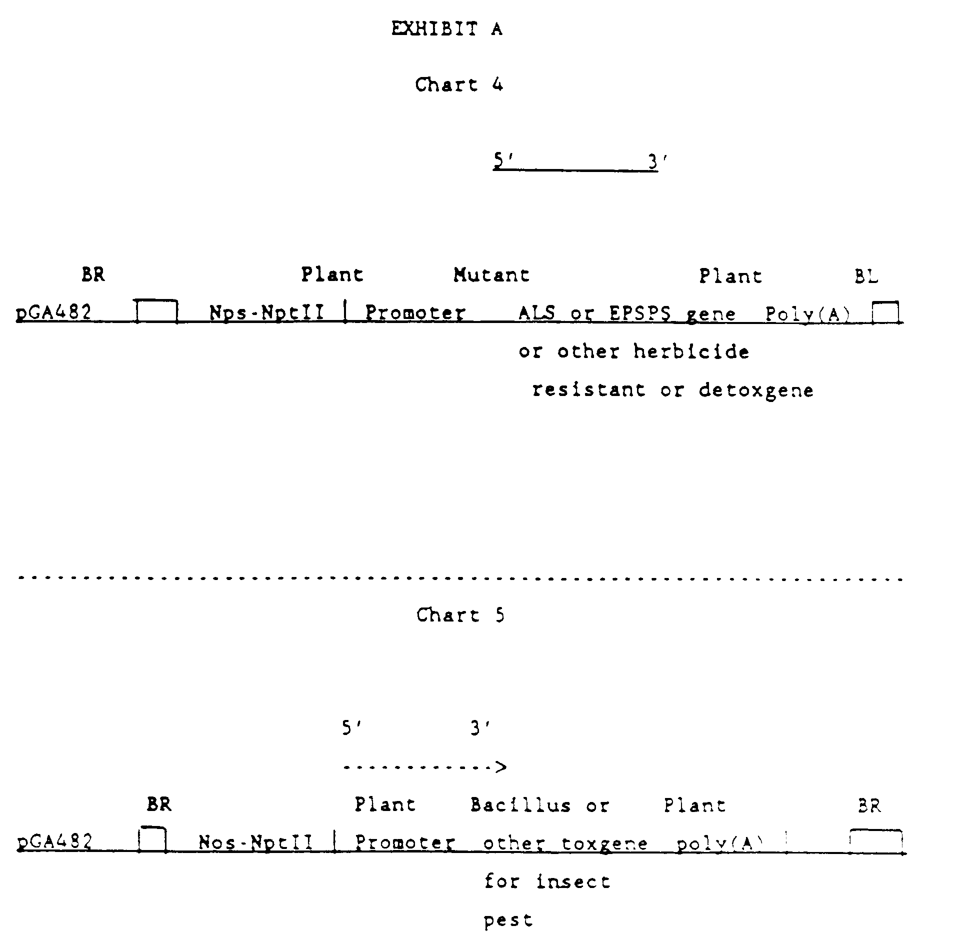
EXHIBIT A

Chart 4

5'          3'

BR                    Plant          Mutant                    Plant          BL
pGA482       Nps-NptII | Promoter    ALS or EPSPS gene    Poly(A)
                                                  or other herbicide
                                                   resistant or detoxgene

Chart 5

5'          3'
------------>
BR                    Plant     Bacillus or     Plant                    BR
pGA482       Nos-NptII | Promoter    other toxgene    poly(A)   
                                                  for insect
                                                  pest

Chart 1

Cmvccoat.Seq Length: 1423 December 1, 1987 12:49 Check: 215
1 GAATTCCCGC CGCAATCGGG AGTTCTTCCG CGTCCCGCTC CGAAGCCTTC
51 AGACCGCAGG TGGTTAACGG TCTTTAGCAC TTTGGTGCGT ATTAGTATAT
101 AAGTATTTGT GAGTCTGTAC ATAATACTAT ATCTATAGAG TCCTGTGTGA
151 GTTGATACAG TAGACATCTG TGACGCGATG CCGTGTTGAG AAGGGATCAC
201 ATCTGGTTTT AGTAAGCCTA CATCATAGTT TTGAGGTTCA ATTCCTCTTA
251 CTCCCTGTTG AGTACCTTAC TTTCTCATGG ATGCTTCTCC GCGAGATTGC
301 GTTATTGTCT ACTGACTATA TAGAGAGTGT GTGTGCTGTG TTTTCTCTTT
351 TGTGTCGTAG AATTGAGTCG AGTCATGGAC AAATCTGAAT CAACCAGTGC
401 TGGTCGTAAC CATCGACGTC GTCCGCGTCG TGGTTCCCGC TCCGCCCCCT
451 CCTCCGCGGA TGCTAACTTT AGAGTCTTGT CGCAGCAGCT TTCGCGACTT
501 AATAAGACGT TAGCAGCTGG TCGTCCAACT ATTAACCACC CAACCTTTGT
551 AGGGAGTGAA CGCTGTAGAC CTGGGTACAC GTTCACATCT ATTACCCTAA
601 AGCCACCAAA AATAGACCGT GAGTCTTATT ACGGTAAAAG GTTGTTACTA
651 CCTGATTCAG TCACGGAATA TGATAAGAAG CTTGTTTCGC GCATTCAAAT
701 TCGAGTTAAT CCTTTGCCGA AATTTGATTC TACCGTGTGG GTGACAGTCC
751 GTAAAGTTCC TGCCTCCTCG GACTTATCCG TTGCCGCCAT CTCTGCTATG
801 TTCGCGGACG GAGCCTCACC GGTACTGGTT TATCAGTATG CCGCATCTGG
851 AGTCCAAGCC AACAACAAAC TGTTGTTTGA TCTTTCGGCG ATGCGCGCTG
901 ATATAGGTGA CATGAGAAAG TACGCCGTCC TCGTGTATTC AAAAGACGAT
951 GCGCTCGAGA CGGACGAGCT AGTACTTCAT GTTGACATCG AGCACCAACG
1001 CATTCCCACA TCTGGAGTGC TCCCAGTCTG ATTCCGTGTT CCCAGAACCC
1051 TCCCTCCGAT CTCTGTGGCG GGAGCTGAGT TGGCAGTTCT ACTACAAACT
1101 GTCTGGAGTC ACTAAACGTT TTACGGTGAA CGGGTTGTCC ATCCAGCTTA
1151 CGGCTAAAAT GGTCAGTCGT GGAGAAATCC ACGCCAGCAG ATTTACAAAT
1201 CTCTGAGGCG CCTTTGAAAC CATCTCCTAG GTTTCTTCGG AAGGGCTTCG
1251 GTCCGTGTAC CTCTAGCGCA ACGTGCTAGT TTCAGGGTAC GGGTGCCCCC
1301 CCACTTTCGT GGGGGCCTCC AAAAGGAGAC CAAAAAAAAA AAAAAAAAAA
1351 AAAAAAAAAA AAAAAAAAAA AAAAAAAAAA AAAAAAAAAA AAAAAAAAAA
1401 AAAAAAAAAA AAAAAAAGAA TTC

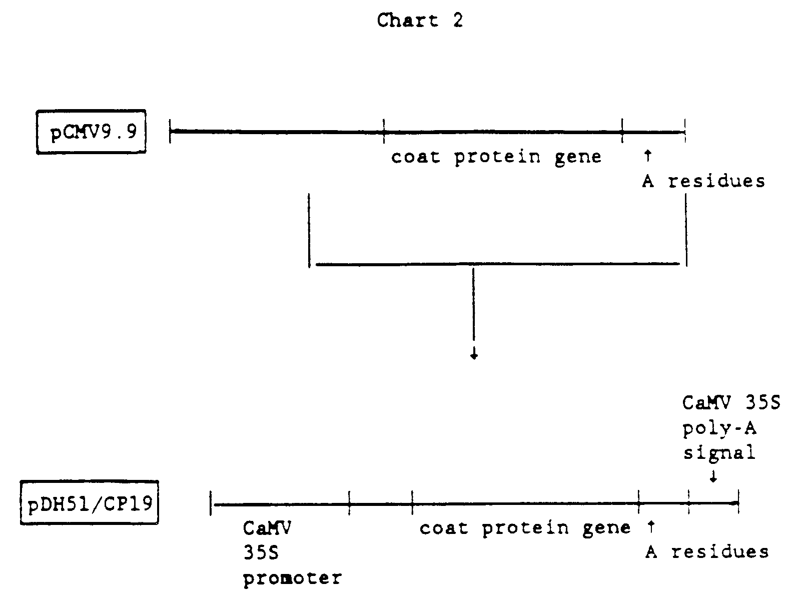
Chart 2

pCMV9.9
coat protein gene
↑
A residues
CaMV 35S
poly-A
signal
↓
pDH51/CP19
CaMV
35S
promoter
coat protein gene
↑
A residues

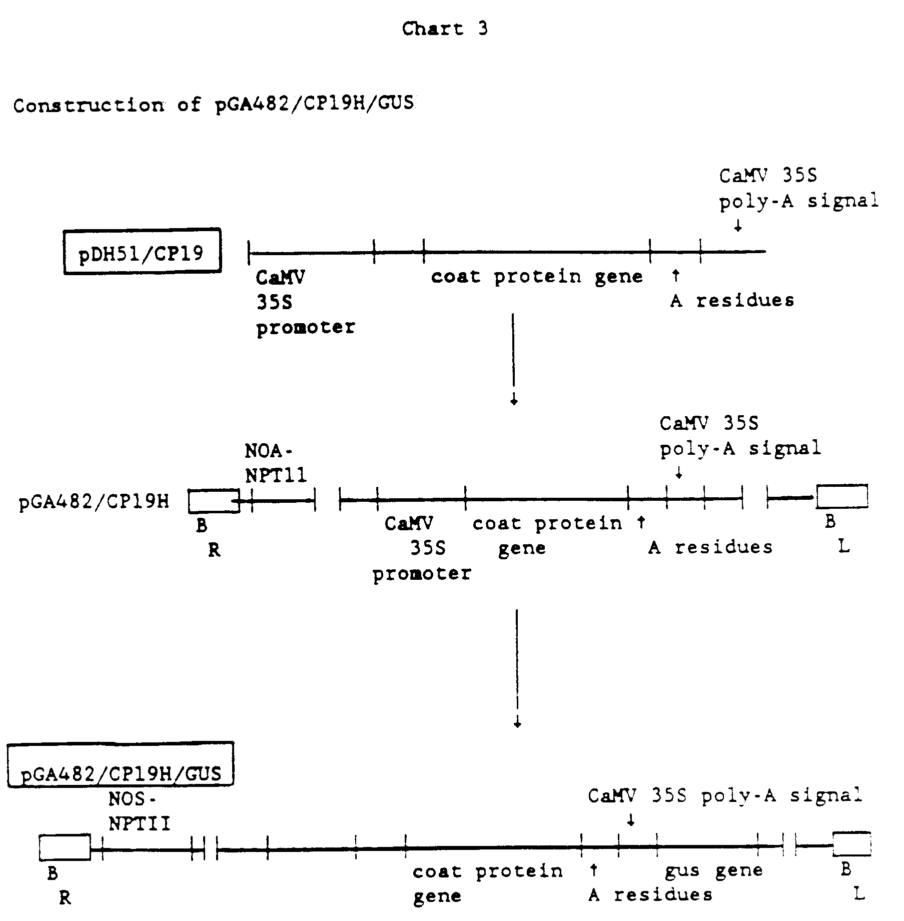
Chart 3

Construction of pGA482/CP19H/GUS

CaMV 35S
poly-A signal
pDH51/CP19
CaMV
35S
promoter
coat protein gene
A residues

CaMV 35S
poly-A signal
NOA-
NPT11
pGA482/CP19H
B
R
CaMV
35S
promoter
coat protein
gene
A residues
B
L

pGA482/CP19H/GUS
NOS-
NPTII
CaMV 35S poly-A signal
B
R
coat protein
gene
A residues
gus gene
B
L

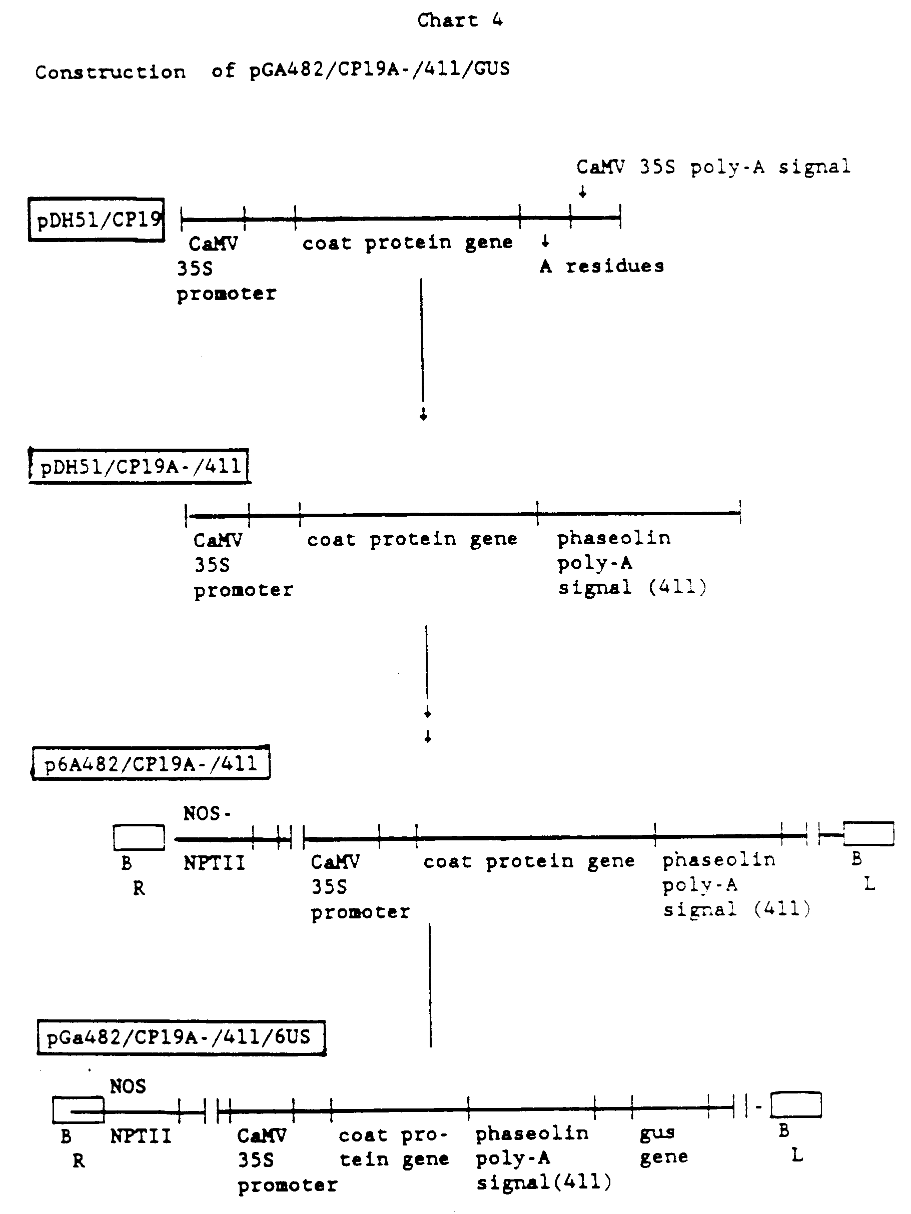
Chart 4

Construction of pGA482/CP19A-/411/GUS

CaMV 35S poly-A signal
pDH51/CP19
CaMV coat protein gene A residues
35S
promoter

pDH51/CP19A-/411
CaMV coat protein gene phaseolin
35S poly-A
promoter signal (411)

p6A482/CP19A-/411
NOS-
B NPTII CaMV coat protein gene phaseolin B
R 35S poly-A L
promoter signal (411)

pGa482/CP19A-/411/6US
NOS
B NPTII CaMV coat pro- phaseolin gus B
R 35S tein gene poly-A gene L
promoter signal(411)

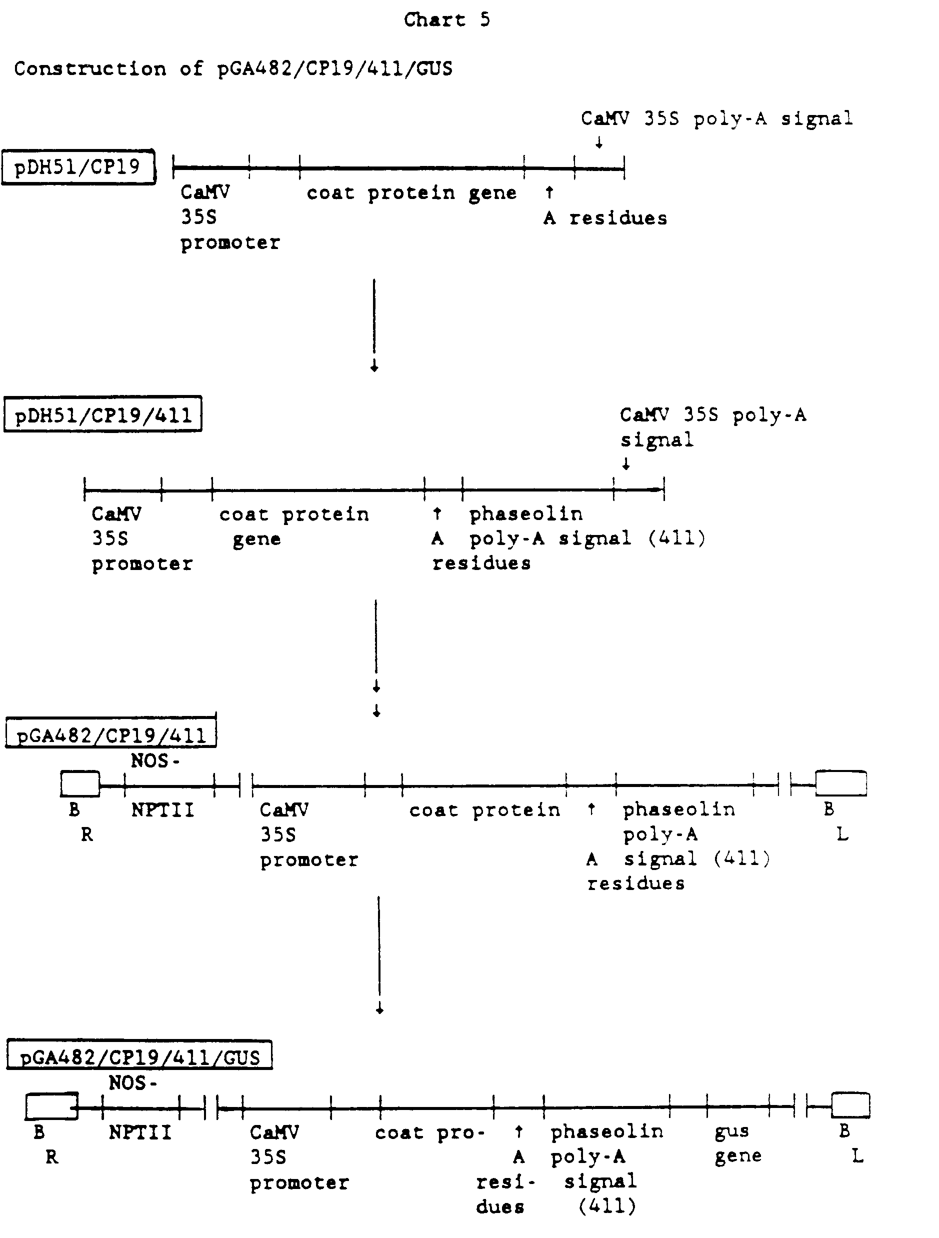
Chart 5

Construction of pGA482/CP19/411/GUS

CaMV 35S poly-A signal
pDH51/CP19
CaMV coat protein gene
35S A residues
promoter

pDH51/CP19/411
CaMV 35S poly-A
signal
CaMV coat protein phaseolin
35S gene A poly-A signal (411)
promoter residues

pGA482/CP19/411
NOS-
B NPTII CaMV coat protein phaseolin B
R 35S poly-A L
promoter A signal (411)
residues

pGA482/CP19/411/GUS
NOS-
B NPTII CaMV coat pro- phaseolin gus B
R 35S A poly-A gene L
promoter resi- signal
dues (411)

## Claims

1. A plant transformation vector comprising the coat protein gene from the C strain of cucumber mosaic virus, having the following sequence: 
1 GAATTCCCGC CGCAATCGGG AGTTCTTCCG CGTCCCGCTC CCAAGCCTTC
51 AGACCGCAGG TCGTTAACGG TCTTTAGTCA CTTTCGTCCG TATTACTATA
101 TAAGTATTTG TGAGTCTGTA CATAATACTA TATCTATAGA GTCCTGTGTG
151 AGTTGATACA GTAGACATCT GTGACGCGAT GCCGTGTTGA GAAGGGATCA
201 CATCTGGTTT TAGTAAGCCT ACATCATAGT TTTGAGGTTC AATTCCTCTT
251 ACTCCCTGTT GAGTACCTTA CTTTCTCATG GATGCTTCTC CGACGAGATT
301 GTCGTTATTG TCTACTGACT ATATAGAGAG TGTGTGTGCT GTGTTTTCTC
351 TTTTGTGTCG TAGAATTGAG TCGAGTCATG GACAAATCTG AATCAACCAG
401 TGCTGGTCGT AACCATCGAC GTCGTCCGCG TCGTGGTTCC CGCTCCGCCC
451 CCTCCTCCGC GGATGCTAAC TTTAGAGTCT TGTCGCAGCA GCTTTCGCGA
501 CTTAATAAGA CGTTAGCAGC TGGTCGTCCA ACTATTAACC ACCCAACCTT
551 TGTAGGGAGT GAACGCTGTA GACCTAGGTA CACGTTCACA TCTATTACCC
601 TAAAGCCACC AAAAATACAC CGTGAGTCTT ATTACGGTAA AACGTTGTTA
651 CTACCTGATT CAGTCACGGA ATATGATAAG AAGCTTGTTT CGCGCATTCA
701 AATTCGAGTT AATCCTTTGC CGAAATTTGA TTCTACCGTG TGGGTGACAG
751 TCCGTAAAGT TCCTGCCTCC TCGGACTTAT CCGTTGCCGC CATCTCTGCT
801 ATGTTCGCGG ACGGAGCCTC ACCGGTACTG GTTTATCAGT ATGCCGCATC
851 TGGAGTCCAA GCCAACAACA AACTGTTGTT TGATCTTTCG GCGATGCGCG
901 CTGATATAGG TGACATGAGA AAGTACGCCG TCCTCGTGTA TTCAAAAGAC
951 GATGCCCTCG AGACGGACGA GCTAGTACTT CATGTTGACA TCCACCACCA
1001 ACGCATTCCC ACATCTGGAG TGCTCCCAGT CTGATTCCGT GTTCCCAGAA
1051 CCCTCCCTCC GATCTCTGTG GCGGGAGCTG AGTTGGCAGT TCTACTACAA
1101 ACTGTCTGCA GTCACTAAAC GTTTTACGGT GAACCGGTTG TCCATCCAGC
1151 TTACGGCTAA AATGGTCAGT CGTGGAGAAA TCCACGCCAG CAGATTTACA
1201 AATCTCTGAG GCGCCTTTGA AACCATCTCC TAGGTTTCTT CGGAAGGGCT
1251 TCGGTCCGTG TACCTCTAGC GCAACGTGCT AGTTTCACGG TACGGGTGCC
1301 CCCCCACTTT CGTGCGGGCC TCCAAAAGGA GACCAAAAAA AAAAAAAAAA
1351 AAAAAAAAAA AAAAAAAAAA AAAAAAAAAA AAAAAAAAAA AAAAAAAAAA
1401 AAAAAAAAAA AAAAAAAAAA GAATTC
 the 35S promoter of cauliflower mosaic virus; and the polyadenylation signal of either the cauliflower mosaic 35S gene or the Phaseolus vulgaris seed storage gene.

2. A bacterial cell containing the plant transformation vector of claim 1.

3. A transformed plant cell containing the gene, the promoter and the polyadenylation signal as defined in claim 1.

4. A cucurbitaceae plant comprising transformed cells according to claim 3.

5. A solanaceae plant comprising transformed cells according to claim 3.

6. A process for producing a virus-resistant cucurbitaceae plant, comprising propagating a cucurbitaceae plant expressing the coat protein gene defined in claim 1.

7. A process for producing a virus-resistant solanaceae plant, comprising propagating a solanaceae plant expressing the coat protein gene defined in claim 1.

## Patentansprüche

1. Pflanzentransformationsvektor mit dem Hülleproteingen des C-Stamms von Gurkenmosaikvirus folgender Sequenz: 
1 CAATTCCCGC CCCAATCGGG AGTTCTTCCG CGTCCCCCTC CCAAGCCTTC
51 ACACCCCACG TCGTTAACGG TCTTTAGTCA CTTTCGTCCG TATTACTATA
101 TAAGTATTTG TGAGTCTCTA CATAATACTA TATCTATACA GTCCTGTCTG
151 AGTTGATACA GTAGACATCT GTGACGCCAT GCCGTGTTGA GAAGGGATCA
201 CATCTGCTTT TAGTAAGCCT ACATCATAGT TTTGAGGTTC AATTCCTCTT
251 ACTCCCTGTT GAGTACCTTA CTTTCTCATG GATGCTTCTC CGACGACATT
301 GTCGTTATTG TCTACTCACT ATATACACAG TCTGTGTGCT GTGTTTTCTC
351 TTTTGTGTCG TAGAATTGAG TCGAGTCATG GACAAATCTG AATCAACCAG
401 TGCTGGTCGT AACCATCGAC GTCGTCCGCG TCCTGGTTCC CGCTCCGCCC
451 CCTCCTCCGC GGATGCTAAC TTTACAGTCT TGTCGCAGCA GCTTTCCCGA
501 CTTAATAAGA CGTTAGCAGC TGGTCGTCCA ACTATTAACC ACCCAACCTT
551 TGTACGGAGT GAACGCTGTA GACCTAGGTA CACGTTCACA TCTATTACCC
601 TAAACCCACC AAAAATACAC CGTCAGTCTT ATTACGGTAA AACCTTGTTA
651 CTACCTGATT CAGTCACGGA ATATGATAAG AACCTTGTTT CGCGCATTCA
701 AATTCCAGTT AATCCTTTGC CGAAATTTGA TTCTACCGTG TGGGTGACAG
751 TCCGTAAAGT TCCTGCCTCC TCGGACTTAT CCGTTGCCGC CATCTCTGCT
801 ATGTTCGCGG ACGGAGCCTC ACCGGTACTG GTTTATCAGT ATGCCGCATC
851 TGGAGTCCAA GCCAACAACA AACTGTTGTT TGATCTTTCG GCGATGCGCG
901 CTGATATAGG TGACATGAGA AAGTACGCCG TCCTCGTGTA TTCAAAAGAC
951 GATGCGCTCG AGACGGACCA GCTAGTACTT CATGTTGACA TCGACCACCA
1001 ACGCATTCCC ACATCTGGAG TGCTCCCAGT CTGATTCCGT GTTCCCAGAA
1051 CCCTCCCTCC GATCTCTGTG GCGGGAGCTG AGTTGGCAGT TCTACTACAA
1101 ACTGTCTGCA GTCACTAAAC GTTTTACGGT GAACGCGTTG TCCATCCAGC
1151 TTACGGCTAA AATGGTCAGT CGTCGACAAA TCCACGCCAG CACATTTACA
1201 AATCTCTGAG GCGCCTTTGA AACCATCTCC TACGTTTCTT CGCAAGCGCT
1251 TCGGTCCGTC TACCTCTAGC GCAACGTGCT AGTTTCACGG TACGCGTGCC
1301 CCCCCACTTT CGTGCGGCCC TCCAAAGGCA GACCAAAAAA AAAAAAAAAA
1351 AAAAAAAAAA AAAAAAAAAA AAAAAAAAAA AAAAAAAAAA AAAAAAAAAA
1401 AAAAAAAAAA AAAAAAAAAA GAATTC
 dem 35S-Promotor von Blumenkohlmosaikvirus und dem Polyadenylierungssignal entweder des Blumenkohlmosaik-35S-Gens oder des Phaseolus vulgaris Samenlagerungsgens.

2. Bakterienzelle mit dem Pflanzentransformationsvektor gemäß Anspruch 1.

3. Transformierte Pflanzenzelle mit dem Gen, dem Promotor und dem Polyadenylierungssignal gemäß Anspruch 1.

4. Cucurbitaceae-Pflanze mit transformierten Zellen nach Anspruch 3.

5. Solanaceae-Pflanze mit transformierten Zellen nach Anspruch 3.

6. Verfahren zur Produktion einer virusresistenten Cucurbitaceae-Pflanze durch Vermehrung einer das Hülleproteingen nach Anspruch 1 exprimierenden Cucurbitaceae-Pflanze.

7. Verfahren zur Produktion einer virusresistenten Solanaceae-Pflanze durch Vermehrung einer das Hülleproteingen nach Anspruch 1 exprimierenden Solanaceae-Pflanze.

## Revendications

1. Vecteur de transformation de végétal, comprenant le gène de la protéine d'enveloppe de la souche C du virus de la mosaïque du concombre, ayant la séquence suivante : 
1 GAATTCCCGC CGCAATCGGG AGTTCTTCCG CGTCCCCCTC CCAAGCCTTC
51 ACACCGCAGG TCGTTAACGG TCTTTAGTCA CTTTGGTCCG TATTACTATA
101 TAAGTATTTG TGAGTCTGTA CATAATACTA TATCTATACA GTCCTGTGTG
151 AGTTGATACA GTAGACATCT GTGACGCGAT GCCGTGTTGA GAAGGGATCA
201 CATCTGCTTT TAGTAAGCCT ACATCATAGT TTTGAGGTTC AATTCCTCTT
251 ACTCCCTCTT GAGTACCTTA CTTTCTCATG GATGCTTCTC CGACGAGATT
301 GTCGTTATTG TCTACTGACT ATATACACAG TGTGTGTCCT GTGTTTTCTC
351 TTTTCTGTCG TAGAATTGAG TCGAGTCATG GACAAATCTG AATCAACCAG
401 TGCTGGTCGT AACCATCGAC GTCGTCCGCG TCCTCGTTCC CGCTCCGCCC
451 CCTCCTCCGC GGATGCTAAC TTTAGAGTCT TGTCGCAGCA GCTTTCGCGA
501 CTTAATAAGA CGTTAGCAGC TGGTCGTCCA ACTATTAACC ACCCAACCTT
551 TGTAGGGAGT GAACGCTGTA GACCTAGGTA CACGTTCACA TCTATTACCC
601 TAAAGCCACC AAAAATAGAC CGTGAGTCTT ATTACGGTAA AAGGTTGTTA
651 CTACCTGATT CAGTCACGGA ATATGATAAG AAGCTTGTTT CGCCCATTCA
701 AATTCCAGTT AATCCTTTGC CGAAATTTGA TTCTACCCTG TGCGTGACAG
751 TCCGTAAAGT TCCTGCCTCC TCGGACTTAT CCGTTGCCGC CATCTCTGCT
801 ATGTTCGCCG ACGGAGCCTC ACCGGTACTG GTTTATCAGT ATGCCGCATC
851 TGGAGTCCAA GCCAACAACA AACTGTTGTT TGATCTTTCG GCGATGCGCG
901 CTCATATAGG TGACATGAGA AAGTACGCCG TCCTCGTCTA TTCAAAAGAC
951 GATGCCCTCG AGACGGACGA GCTAGTACTT CATGTTGACA TCGAGCACCA
1001 ACGCATTCCC ACATCTGGAG TGCTCCCAGT CTGATTCCGT GTTCCCAGAA
1051 CCCTCCCTCC GATCTCTGTG GCGGCACCTG AGTTCCCAGT TCTACTACAA
1101 ACTGTCTGCA GTCACTAAAC GTTTTACGGT GAACGGGTTG TCCATCCAGC
1151 TTACGGCTAA AATGGTCAGT CGTGGAGAAA TCCACGCCAG CAGATTTACA
1201 AATCTCTGAG GCGCCTTTGA AACCATCTCC TAGGTTTCTT CGCAACCCCT
1251 TCGGTCCGTG TACCTCTAGC GCAACGTGCT AGTTTCACGG TACGGTGCC
1301 CCCCCACTTT CGTGGGGGCC TCCAAAGGA GACCAAAAAA AAAAAAAAAA
1351 AAAAAAAAAA AAAAAAAAAA AAAAAAAAAA AAAAAAAAAA AAAAAAAAAA
1401 AAAAAAAAAA AAAAAAAAAA GAATTC
 Le promoteur 35S du virus de la mosaïque du chou-fleur ; et le signal de polyadénylation du gène 35S de la mosaïque du chou-fleur ou le gène de stockage des graines de Phaseolus vulgaris.

2. Cellule bactérienne contenant le vecteur de transformation de végétal suivant la revendication 1.

3. Cellule végétale transformée contenant le gène, le promoteur et le signal de polyadenylation définis dans la revendication 1.

4. Plante de la famille des cucurbitacées, comprenant des cellules transformées suivant la revendication 3.

5. Plante de la famille des solanacées, comprenant des cellules transformées suivant la revendication 3.

6. Procédé de production d'une plante de la famille des cucurbitacées, résistant aux virus, comprenant la multiplication d'une plante de la famille des cucurbitacées exprimant le gène de protéine d'enveloppe défini dans la revendication 1.

7. Procédé de production d'une plante de la famille des solanacées, résistant aux virus, comprenant la multiplication d'une plante de la famille des solanacées exprimant le gène de protéine d'enveloppe défini dans la revendication 1.
